# EUROPEAN PATENT APPLICATION

(11) **EP 4 659 794 A1**
(43) Date of publication of application: **10.12.2025**
(21) Application number: 24750406.1
(22) Date of filing: 02.02.2024
(51) Int. Cl.: A61M 37/00, A61M 5/142, H02J 50/10

(54) **POWER FEEDING DEVICE AND MEDICAL DEVICE**

(30) Priority: 03.02.2023 JP 2023015311
(71) Applicant: FURUKAWA ELECTRIC CO., LTD., Tokyo 100-8322 (JP)
(72) Inventor: NOMURA Yoshiki, Tokyo 100-8322 (JP); OCHIAI Makoto, Tokyo 100-8322 (JP); HASEGAWA Junichi, Tokyo 100-8322 (JP)
(74) Representative: Winter, Brandl - Partnerschaft mbB
(86) International application number: PCT/JP2024/003414
(87) International publication number: WO 2024/162457

(57) **Abstract**

Provided is a power feeding device capable of easily removing from the surface of a living body in a state where an injection needle is inserted in a lid of a medical instrument embedded in the living body. A power feeding device 20 for feeding power to a medical instrument 10 in a non-contact manner comprises a power transmission coil 22 that is formed in an annular shape and has a conductor extending along the annular shape. The power transmission coil 22 has a connector 22a1 that functions as a separation/connection part which is connected to the conductor so as to be separable and from which a prescribed portion of the annular shape is separated together with the conductor.

## Description

### TECHNICAL FIELD

The present invention relates to a power feeding device and a medical device for feeding power to target equipment in a non-contact manner.

### BACKGROUND ART

Conventionally, a power feeding device for feeding power to a medical instrument embedded in a living body in a non-contact manner is known, the power feeding device including a body part including a power supply, and a power transmission coil that is formed in an annular shape and has a conductor extending along the annular shape (see, for example, Patent Document 1).

The medical instrument includes a body part having a recess capable of storing a chemical solution, a lid including a soft member that closes the recess of the body part, a power reception coil that receives power sent from the power feeding device, and a light emitter that is placed along an outer peripheral side of the lid and emits by the power received with the power reception coil.

The power feeding device feeds power to the medical instrument with the power transmission coil brought close to the medical instrument embedded in the living body. The medical instrument that has received the fed power irradiates the surface of the living body with light emitted from the light emitter and directed from the outer peripheral side of the lid and notifies the position of the lid in the living body. When injecting the chemical solution into the recess of the body part of the medical instrument, an injection needle is inserted in the lid from outside the living body through an inner circumferential side of the annular shape of the power transmission coil while holding a state where the position of the lid is notified by the light emitter, to inject the chemical solution into the recess.

### Citation List

### Patent Document

Patent Document 1: Japanese Unexamined Patent Application, Publication No. 2020-182679

### DISCLOSURE OF THE INVENTION

### Problems to be Solved by the Invention

The power feeding device is removed from the surface of a living body by passing an injection needle through a gap formed in a part of the annular shape of a power transmission coil in a state where the injection needle is inserted in a lid of a medical instrument from outside the living body. Consequently, when removing the power feeding device from the surface of the living body in the state where the injection needle is inserted in the lid of the medical instrument from outside the living body, it is not easy to pass the injection needle through a small gap in the power transmission coil.

An object of the present invention is to provide a power feeding device and a medical device that can be easily removed from the surface of a living body in a state where an injection needle is inserted in a lid of a medical instrument embedded in the living body.

### Means for Solving the Problems

A power feeding device according to the present invention is a power feeding device for feeding power to target equipment in a non-contact manner and includes a power transmission coil that is formed in an annular shape and has a conductor extending along the annular shape, and the power transmission coil includes a separation/connection part which is connected to the conductor so as to be separable and from which a prescribed portion of the annular shape is separated together with the conductor.

Furthermore, in the power feeding device according to the present invention, it is preferable that the power transmission coil includes a coil body, and a detachable coil that is configured to be detachably attached to the coil body via the separation/connection part.

Additionally, in the power feeding device according to the present invention, it is preferable that the detachable coil includes a plurality of conductive wires.

In addition, in the power feeding device according to the present invention, it is preferable that the plurality of conductive wires are connected in series by connecting the detachable coil to the coil body.

Furthermore, in the power feeding device according to the present invention, it is preferable that the plurality of conductive wires are connected in parallel by connecting the detachable coil to the coil body.

Additionally, a medical device according to the present invention is a medical device including a medical instrument embedded in a living body, and a power feeding device for feeding power to the medical instrument in a non-contact manner, the power feeding device including a power transmission coil that is formed in an annular shape and has a conductor extending along the annular shape, the power transmission coil including a separation/connection part which is connected to the conductor so as to be separable and from which a prescribed portion of the annular shape is separated together with the conductor.

### Effects of the Invention

According to the present invention, the power transmission coil can have the annular shape converted to a linear shape or can be disassembled into a plurality of members, so that a power feeding device can be easily removed from the surface of the living body in a state where an injection needle is inserted in a lid of a medical instrument embedded in the living body, on an inner circumferential side of the annular shape of the power transmission coil.

### BRIEF DESCRIPTION OF THE DRAWINGS

FIG. 1 is a side cross-sectional view of a medical device according to one embodiment of the present invention;
FIG. 2 is a perspective view of a power feeding device according to one embodiment of the present invention;
FIG. 3 is an exploded perspective view of a part of the power feeding device according to one embodiment of the present invention;
FIG. 4 is an electrical circuit diagram of the power feeding device according to one embodiment of the present invention; and
FIG. 5 is an electrical circuit diagram of a power feeding device according to another embodiment of the present invention.

### PREFERRED MODE FOR CARRYING OUT THE INVENTION

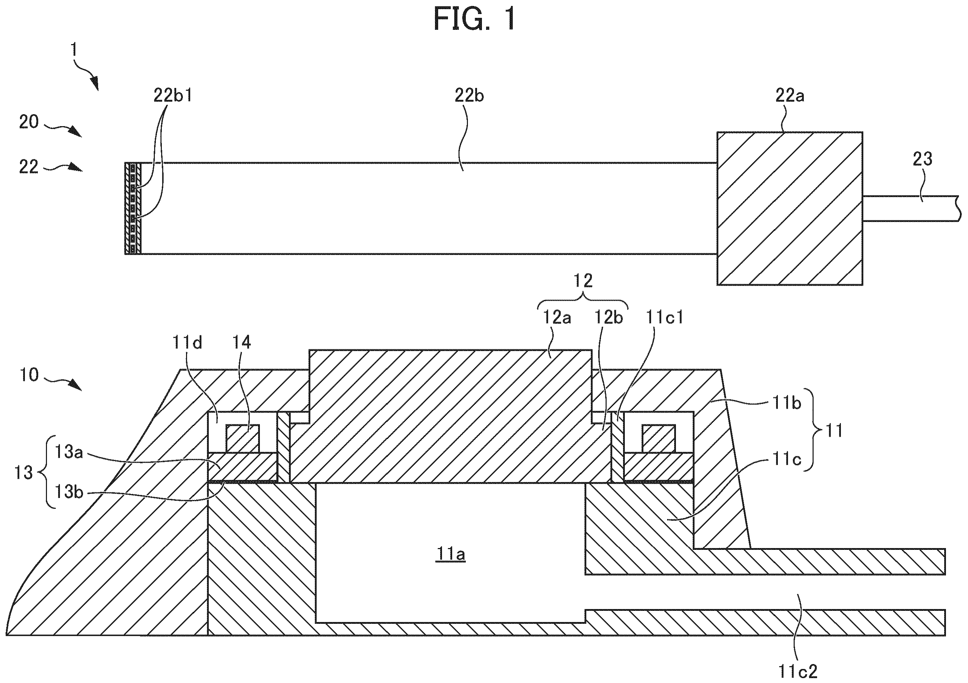

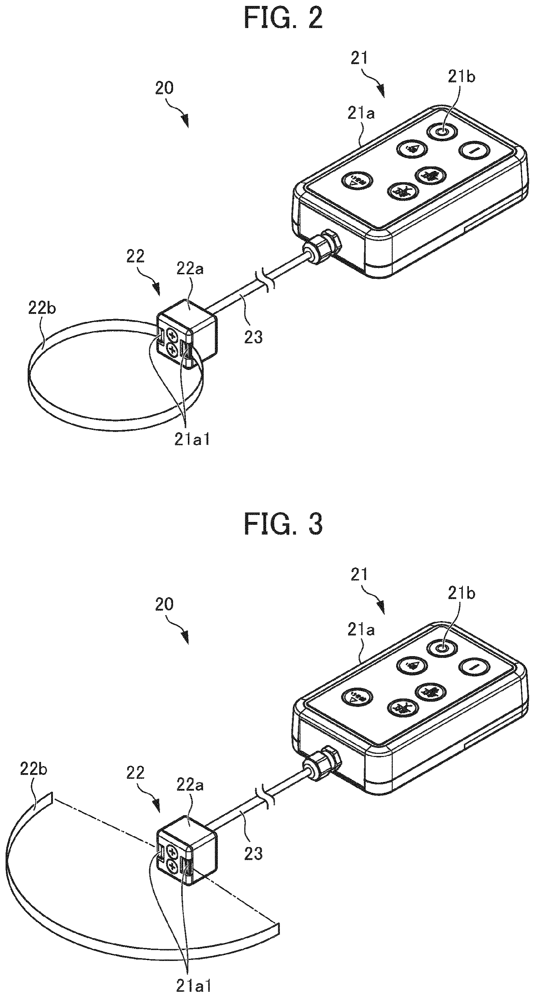

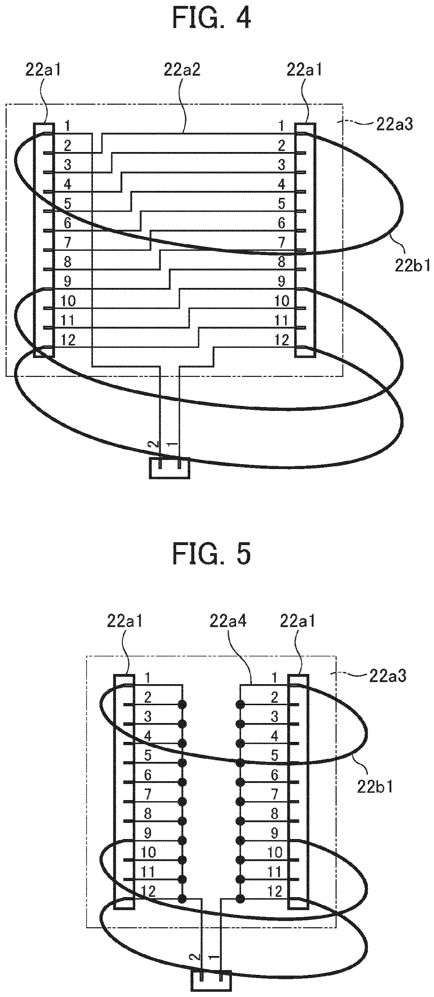

FIGS. 1 to 4 illustrate one embodiment of the present invention. FIG. 1 is a side cross-sectional view of a medical device, FIG. 2 is a perspective view of a power feeding device, FIG. 3 is an exploded perspective view of a part of the power feeding device, and FIG. 4 is an electrical circuit diagram of the power feeding device.

As shown in FIG. 1, a medical device 1 of the present embodiment includes a medical instrument 10 used in a state of being embedded in a living body of a human or an animal, and a power feeding device 20 for feeding power to the medical instrument 10. The medical device 1 feeds power from the power feeding device 20 to the medical instrument 10 in a non-contact manner.

The medical instrument 10 is a subcutaneous implantable port (CV port) that is a type of central venous catheter that injects a chemical solution into a central vein located in the vicinity of the heart of the living body. As shown in FIG. 1, the medical instrument 10 includes a body part 11 having a recess 11a capable of storing a liquid such as the chemical solution, a lid 12 that closes an opening of the recess 11a of the body part 11, a power receiver 13 that receives power sent from the power feeding device 20, a plurality of light emitters 14 that are connected to the power receiver 13 and emit light on an outer peripheral side of the lid 12 by the power received by the power receiver 13, and a catheter (not shown) having one end communicating with the recess 11a of the body part 11, and the other end inserted into the central vein of the living body.

The body part 11 is made, for example, of a resin material such as polyphenylene sulfide (PPS), polyethersulfone (PES), or polyetheretherketone (PEEK), and is formed in a substantially truncated conical shape. In the body part 11, the recess 11a is formed to have an upper surface opened and linearly extend toward a bottom surface side, and liquid is stored in the recess 11a. The body part 11 includes an upper side member 11b and a lower side member 11c, and in a space 11d formed between the upper side member 11b and the lower side member 11c, the power receiver 13 and a light emitter 14 are housed. The space 11d is formed in an annular shape to surround an outer peripheral side of the recess 11a. Furthermore, in an upper surface of the lower side member 11c, a partition wall 11c1 is provided to divide the space 11d into the lid 12, and the power receiver 13 and light emitter 14. In the lower side member 11c, a fluid path 11c2 is provided to extend from a bottom side of the recess 11a toward the outer peripheral side of the recess and formed to allow the liquid stored in the recess 11a to flow therethrough. One end of the catheter is connected to the fluid path 11c2. The recess 11a may be referred to as a chemical solution tank or the like.

The lid 12 is made of a soft member such as silicone rubber through which an injection needle is repeatedly inserted. The lid 12 includes a columnar lid body 12a, and a flange 12b extending outward along a circumferential direction of an outer peripheral portion of the lid body 12a. The lid body 12a is a portion in which the injection needle is repeatedly inserted from the outside of the living body. Furthermore, the flange 12b is a portion located in the space 11d of the body part 11.

The power receiver 13 includes a circuit board 13a, and a power reception coil 13b that receives power from the power feeding device 20.

The circuit board 13a is formed in an annular shape and housed in the space 11d of the body part 11. The power reception coil 13b is connected to the circuit board 13a to configure a resonance circuit (not shown). On the upper surface of the circuit board 13a, a plurality of light emitters 14 connected to the resonance circuit are placed to emit light by the power received by the power reception coil 13b.

Each of the plurality of light emitters 14 is a light emitting diode (LED) that emits light by an applied voltage for example, and the light emitters 14 are arranged at intervals in the circumferential direction on the upper surface of the circuit board 13a of the power receiver 13. Each of the plurality of light emitters 14 has an irradiation direction, which is a light emitting direction, directed upward.

As shown in FIGS. 2 and 3, the power feeding device 20 includes a body part 21 including a power supply, and a power transmission coil 22 that is formed in an annular shape and has a conductor extending along the annular shape.

The body part 21 includes a housing 21a having a size that can be grasped by a person by hand. Inside the housing 21a, a circuit board on which components such as a power supply and an IC chip are mounted is housed. Furthermore, the housing 21a includes a plurality of operation buttons 21b for performing operations of turning on and off the power supply, adjusting the brightness of the light emitter 14, and the like.

The power transmission coil 22 includes a coil body 22a connected to the body part 21 via a cable 23, and a detachable coil 22b provided to be detachably attached to the coil body 22a.

The coil body 22a includes a circuit board 22a3 including connectors 22a1 and 22a1 as a pair of separation/connection parts in which terminals are arranged, and a wiring 22a2 that connects respective terminals of the pair of connectors 22a1 and 22a1 to the power supply. Each of the pair of connectors 22a1 and 22a1 is a flip-lock connector in which 12 terminals are arranged, for example, in a width direction and that can hold a state where the cable is connected.

The detachable coil 22b is a flexible cable formed in a band shape by coating, with an insulator, a plurality of conductive wires 22b1 arranged in the width direction. The detachable coil 22b is, for example, a so-called flexible flat cable including 12 conductors arranged at an interval of 0.5 mm. The detachable coil 22b has each of opposite ends from which the insulator is removed to expose the conductor, the ends being connected to the wiring 22a2 of the coil body 22a via the pair of connectors 22a1 and 22a1.

As shown in FIG. 2, the power transmission coil 22 is formed in an annular shape in a state where the detachable coil 22b is connected to the pair of connectors 22a1 and 22a1 of the coil body 22a. Furthermore, as shown in FIG. 4, the plurality of conductive wires 22b1 of the detachable coil 22b are connected in series in a state where the detachable coil 22b is connected to the pair of connectors 22a1 and 22a1 of the coil body 22a.

In the medical device 1 configured as described above, the medical instrument 10 is used in a state of being embedded in the living body. A liquid such as the chemical solution administered to the living body is injected into the recess 11a of the body part 11 via an injection needle such as a Huber needle in a state where the lid 12 is pierced from the outside of the living body. The liquid injected into the recess 11a of the body part 11 flows in the catheter through the fluid path 11c2 and is administered into the central vein of the living body.

Furthermore, for the medical instrument 10 embedded in the living body, it is necessary to identify the position of the lid 12 in the living body when the injection needle is inserted in the lid 12. Consequently, when the injection needle is inserted in the lid 12 of the medical instrument 10 embedded in the living body, the power transmission coil 22 of the power feeding device 20 is brought closer to a region in which the medical instrument 10 is embedded from the outside of the living body, and electricity is applied to the power transmission coil 22. Consequently, resonance is generated between the power transmission coil 22 and the power reception coil 13b, and power is transmitted from the power transmission coil 22 to the power reception coil 13b, allowing the plurality of light emitters 14 to emit light by the power received by the power reception coil 13b. Each of the plurality of light emitters 14 emits light upward from the body part 11 thereby emitting light upward from the outer periphery of the lid 12, causing light to reach an outer peripheral part of the lid 12 on the surface of the living body, so that the position of the lid 12 can be identified. In this state, the injection needle is inserted through a portion surrounded by the plurality of light emitters 14 via an inner circumferential side of the annular shape of the power transmission coil 22, and the injection needle can be thus reliably inserted in the lid 12.

Removing the power feeding device 20 from the surface of the living body in a state where the injection needle is inserted in the lid 12 of the medical instrument 10 embedded in the living body includes operating one of or both the pair of connectors 22a1 and 22a1, and removing one end or opposite ends of the detachable coil 22b from the coil body 22a of the power transmission coil 22, to separate at least a part of the annular shape. Consequently, the power transmission coil 22 has the annular shape converted to a linear shape or is disassembled into a plurality of forms, so that the power feeding device 20 can be easily removed from the surface of the living body in a state where the injection needle is inserted in the lid 12.

Thus, the power feeding device according to the present embodiment provides the power feeding device 20 for feeding power to the medical instrument 10 as target equipment in a non-contact manner and includes the power transmission coil 22 that is formed in the annular shape and has the conductor extending along the annular shape, and the power transmission coil 22 includes the connector 22a1 as the separation/connection part which is connected to the conductor so as to be separable and from which a prescribed portion of the annular shape is separated together with the conductor.

Furthermore, a medical device according to the present embodiment provides the medical device 1 including the medical instrument 10 embedded in the living body, and the power feeding device 20 for feeding power to the medical instrument 10 in a non-contact manner, the power feeding device 20 including the power transmission coil 22 that is formed in the annular shape and has the conductor extending along the annular shape, the power transmission coil 22 including the connector 22a1 as the separation/connection part which is connected to the conductor so as to be separable and from which a prescribed portion of the annular shape is separated together with the conductor.

Consequently, the power transmission coil 22 can have the annular shape converted to a linear shape or can be disassembled into a plurality of members, so that the power feeding device 20 can be easily removed from the surface of the living body in a state where the injection needle is inserted in the lid 12 of the medical instrument 10 embedded in the living body, on the inner circumferential side of the annular shape of the power transmission coil 22.

Furthermore, the power transmission coil 22 preferably includes the coil body 22a, and the detachable coil 22b configured to be detachably attached to the coil body 22a via the connectors 22a1 and 21a1 as the separation/connection parts.

Consequently, the detachable coil 22b can be replaced for the coil body 22a, so that the size of the annular shape of the power transmission coil 22 can be set to an arbitrary size by adjusting the length of the detachable coil 22b.

Furthermore, it is preferable that the detachable coil 22b includes the plurality of conductive wires 22b1.

Consequently, by attaching the detachable coil 22b to the coil body 22a, a coil having a plurality of turns can be configured, so that operation efficiency can be improved during an assembling operation of the power transmission coil 22.

It is preferable that the plurality of conductive wires 22b1 are connected in series by connecting the detachable coil 22b to the coil body 22a.

Consequently, inductance in the power transmission coil 22 can be increased.

FIG. 5 is an electrical circuit diagram of a power feeding device, showing another embodiment of the present invention. The same components as in the above embodiment are denoted with the same reference numerals.

In a power feeding device 20 of the present embodiment, as shown in FIG. 5, a circuit board 22a3 of a coil body 22a of a power transmission coil 22 includes connectors 22a1 and 22a1 as a pair of separation/connection parts in which terminals are arranged, and a wiring 22a4 that connects respective terminals of the pair of connectors 22a1 and 22a1 to a power supply. In the same manner as in the above embodiment, the power transmission coil 22 of the present embodiment is formed in an annular shape in a state where a detachable coil 22b is connected to the pair of connectors 22a1 and 22a1 of the coil body 22a. Furthermore, a plurality of conductive wires 22b1 of a detachable coil 22b are connected in parallel in a state where the detachable coil 22b is connected to the pair of connectors 22a1 and 22a1 of the coil body 22a.

Consequently, according to the power feeding device and medical device of the present embodiment, as with the above embodiment, the power transmission coil 22 can have the annular shape converted to a linear shape or can be disassembled into a plurality of members, so that the power feeding device 20 can be easily removed from the surface of the living body in a state where the injection needle is inserted in the lid 12 of the medical instrument 10 embedded in the living body, on the inner circumferential side of the annular shape of the power transmission coil 22.

Furthermore, it is preferable that the plurality of conductive wires 22b1 are connected in parallel by connecting the detachable coil 22b to the coil body 22a.

Consequently, reactance in the power transmission coil 22 can be decreased.

Additionally, the above embodiment illustrates that the power transmission coil 22 includes the coil body 22a, and the detachable coil 22b configured to be detachably attached to the coil body 22a and is not limited thereto. In the power transmission coil, at least a part of the annular shape may only be separable via the separation/connection part. The annular shape of the power transmission coil may be converted to a linear shape, for example, by forming the power transmission coil in entirety in the annular shape by a flexible flat cable, providing the separation/connection part at one location of the annular shape, and dividing the annular shape in the separation/connection part. Alternatively, for example, a mechanism may be configured in which a gap that is a separation/connection part can be formed at one location of the annular shape of the power transmission coil, to form the gap in a part of the annular shape by a predetermined operation.

Furthermore, the above embodiment illustrates that the flexible flat cable is used as the detachable coil 22b and is not limited thereto. The detachable coil 22b may only include, for example, a plurality of conductors insulated from each other, such as a multi-core cable, a ribbon cable, and is a flexible printed circuit board. The number of the conductors of the detachable coil may be set depending on the number of turns required for the power transmission coil, and preferably 8 or more and 25 or less. Furthermore, it is preferable that the detachable coil has a length of 15 cm or more, and 40 cm or less.

In addition, the above embodiment illustrates that the body part 21 and the power transmission coil 22 are connected by the cable 23, and the body part and power transmission coil may be integrally formed via no cable.

Furthermore, the above embodiment illustrates the power feeding device 20 for feeding power to the medical instrument 10 embedded in the living body, and is not limited to the medical instrument, and the present invention can be applied to equipment including a power reception coil that receives power in a non-contact manner.

Additionally, the above embodiment illustrates the flip-lock connector 22a1 as the separation/connection part and is not limited thereto. The separation/connection part may be selected depending on the type of detachable coil, and for example, in addition to the flip-lock connector, the separation/connection part may be a back flip-lock connector, an insulation displacement connector, a crimp connector, or an insertion connector. Furthermore, the shape of the connector as the separation/connection part may be round in addition to a rectangular shape and may be waterproof.

### EXPLANATION OF REFERENCE NUMERALS

1 medical device
10 medical instrument
20 power feeding device
22 power transmission coil
22a coil body
22a1 connector
22b detachable coil
22b1 conductive wire

## Claims

1. A power feeding device for feeding power to target equipment in a non-contact manner, comprising
a power transmission coil that is formed in an annular shape and has a conductor extending along the annular shape,
the power transmission coil including a separation/connection part which is connected to the conductor so as to be separable and from which a prescribed portion of the annular shape is separated together with the conductor.

2. The power feeding device according to claim 1, wherein
the power transmission coil includes a coil body, and a detachable coil that is configured to be detachably attached to the coil body via the separation/connection part.

3. The power feeding device according to claim 2, wherein
the detachable coil includes a plurality of conductive wires.

4. The power feeding device according to claim 3, wherein
the plurality of conductive wires are connected in series by connecting the detachable coil to the coil body.

5. The power feeding device according to claim 3, wherein
the plurality of conductive wires are connected in parallel by connecting the detachable coil to the coil body.

6. A medical device comprising:
a medical instrument embedded in a living body; and
a power feeding device for feeding power to the medical instrument in a non-contact manner,
the power feeding device including a power transmission coil that is formed in an annular shape and has a conductor extending along the annular shape,
the power transmission coil including a separation/connection part which is connected to the conductor so as to be separable and from which a prescribed portion of the annular shape is separated together with the conductor.
